# EUROPEAN PATENT SPECIFICATION

(11) **EP 2 611 453 B1**
(45) Date of publication and mention of the grant of the patent: **18.08.2021**
(21) Application number: 11822182.9
(22) Date of filing: 29.08.2011
(51) Int. Cl.: A61K 35/64, A61K 38/40, A61K 31/05, A61K 36/61, A61P 17/02, A61K 31/70, A61K 38/02, A61P 31/10

(54) **ANTIFUNGAL COMPOSITION**
ANTIMYKOTISCHE ZUSAMMENSETZUNG
COMPOSITION ANTIFONGIQUE

(30) Priority: 30.08.2010 NZ 58764210
(43) Date of publication of application: 10.07.2013
(73) Proprietor: Comvita New Zealand Limited, Te Puke (NZ)
(72) Inventor: GANNABATHULA, Swapna, Auckland (NZ); STEPHENS, Jonathan, McDonald Counsell, Hamilton (NZ)
(74) Representative: Mewburn Ellis LLP
(86) International application number: PCT/NZ2011/000172
(87) International publication number: WO 2012/030231

(56) References cited:
- WO-A1-2005/085287
- WO-A1-2007/137881
- WO-A1-2010/082846
- WO-A2-2008/023165
- WO-A2-2008/049578
- RU-C1- 2 220 708
- LUSBY ET AL: "Bactericidal Activity of Different Honeys against Pathogenic Bacteria", ARCHIVES OF MEDICAL RESEARCH, INSTITUTO MEXICANO DEL SEGURO SOCIAL, MEXICO, MX, vol. 36, no. 5, 1 September 2005 (2005-09-01), pages 464-467, XP005024627, ISSN: 0188-4409
- BRADY N ET AL: "A survey of non-manuka New Zealand honeys for antibacterial and antifungal activities", JOURNAL OF APICULTURAL RESEARCH, vol. 43, no. 2, 2004, pages 47-52, XP009175382, ISSN: 0021-8839
- AL-JABRI A.A. ET AL.: 'Honey, milk and antibiotics' AFRICAN JOURNAL OF BIOTECHNOLOGY vol. 4, no. 13, December 2005, pages 1580 - 1587, XP055077182
- None

## Description

### TECHNICAL FIELD

The application relates to an antifungal composition. More specifically, the application relates to a composition for use in the therapeutic inhibition of yeast growth including a combination of lactoferrin and either honey, a honey extract or a honey analogue.

### BACKGROUND ART

Honey is well known and discussed in the art for its antimicrobial effects. Honey inhibits microbial growth due to a variety of factors, all of which overwhelm the microbe defence mechanisms. Examples of anti-microbial factors include the osmolarity of honey (and resulting low water activity), the low pH of honey, the presence of hydrogen peroxide, the presence of bee defensins and other compounds such as methylglyoxal (MGO) and phenolic compounds. Fungal treatments using honey are less widely documented although it is known that honey has antifungal properties.

Metal chelators including lactoferrin are well known in the art. Chelators are essentially molecules that react quickly with free hydrogen or metal ions binding up the ions within the molecule and removing them from a solution. Early descriptions of chelators likened a chelator to being a crab's claw (the molecular array) surrounding the ion (e.g., a metal ion) and not allowing the ion to react with other molecules. Chelators can have very important physiological effects. In particular, they help the body regulate the presence of trace metals in the body, both preventing an overdose as well as providing a reservoir of metal ions for use when trace metals become low. Haemoglobin is one important metal (iron) chelator in the body that ensures stocks of iron remain present as it is an essential element in normal metabolism. Metal ions in general are important cofactors to many biological reactions and an overabundance of chelating compounds essentially starves the normal metabolic process.

Metals that may be chelated include the transition metals including calcium, magnesium, iron, copper and zinc.

Known metal chelators are varied and include (but are not limited to) lactoferrin, shellfish extracts (including those from green lipped mussel and oysters) particularly those containing cavortin protein, nut tannins, seaweeds, green tea, phytic acid, potato peel and many others.

Existing metal chelators are known to deprive microbes of essential minerals required for growth. For example iron is a necessary trace mineral for haemoglobin enzyme activity.

Combinations of honey, a honey fraction or a honey analogue and lactoferrin that act synergistically against yeast growth are not in the inventor's experience known in the art and it should be appreciated that synergism against yeast infection would be advantageous.

For the purpose of this specification, and unless otherwise noted, the term 'comprise' and grammatical variations thereof shall have an inclusive meaning - i.e. that it will be taken to mean an inclusion of not only the listed components it directly references, but also other non-specified components or elements.

WO 2005/085287 describes a method of preparing an ultra-cleansed lactoferrin preparation, termed treatment for contaminant reduction (TCR) which includes the steps of treating commercial lactoferrin preparation with at least one each of surfactants, antioxidants and polyphenols to form purified lactoferrin (LF-TCR) and drying the LF-TCR. Additionally a therapeutic lactoferrin composition is described which contains LF-TCR and optionally surfactants, antioxidants, polyphenols, tissue/membrane diffusion facilitating agents and anionic compounds.

WO 2008/023165 describes a composite material comprising lactoferrin and a bioactive glass. It also describes pharmaceutical compositions containing the composite material and the use of the composite material for treating a wound, treating or preventing bacterial or viral infections in a wound, preventing viral transmission, regenerating bone, treating osteoporosis, preventing or alleviating bleeding in a wound, sterilising a wound and/or controlling haemorrhaging.

LUSBY ET AL: "Bactericidal Activity of Different Honeys against Pathogenic Bacteria", ARCHIVES OF MEDICAL RESEARCH, INSTITUTO MEXICANO DEL SEGURO SOCIAL, MEXICO, MX, vol. 36, no. 5, 1 September 2005, pages 464-467, describes an assessment of the antibacterial effect of various honeys. An agar dilution method was used to assess the activity of honeys against 13 bacteria and one yeast. The honeys were tested at five concentrations ranging from 0.1 to 20%. Twelve of the 13 bacteria were inhibited by all honeys used in this study with only *Serratia marcescens* and the yeast *Candida albicans* not inhibited by the honeys. Little or no antibacterial activity was seen at honey concentrations < 1 %, with minimal inhibition at 5%. No honey was able to produce complete inhibition of bacterial growth.

BRADY N ET AL: "A survey of non-manuka New Zealand honeys for antibacterial and antifungal activities", JOURNAL OF APICULTURAL RESEARCH, vol. 43, no. 2, 2004, pages 47-52, describes a study using 179 unifloral, unpasteurized honey samples obtained from commercial beekeepers throughout New Zealand. The honeys were tested against *Staphylococcus aureus* and *Candida albicans, Escherichia* coli and the dermatophyte *Trichophyton mentagrophytes* using agar well diffusion, measurement of minimum inhibitory concentration and modified agar well diffusion methods, respectively. Of the 179 non-manuka honey samples assessed, none showed non-peroxide or anti-yeast antimicrobial activities. By contrast, 50% of the samples tested showed antibacterial activity against *S. aureus,* with activity ranging from 5.0-27.9% phenol equivalent. Approximately 30% of the samples tested showed antibacterial activity against *E. coli;* however honey concentrations required for inhibition were with one exception, in excess of 19%. Similarly, 35% of samples showed antifungal activity although the levels of activity measured were low.

### SUMMARY

The application broadly relates to the combination of honey, an active fraction of honey or a honey analogue with lactoferrin as a means to inhibit the growth of yeasts.

In a first aspect there is provided a composition for use in the therapeutic inhibition of yeast growth, the composition including:
a. a therapeutically effective amount of lactoferrin;
b. a therapeutically effective amount of either:
   (i) at least one active honey; or
   (ii) at least one bioactive fraction of active honey wherein the monosaccharides of the honey have been removed; or
   (iii) at least one honey analogue containing monosaccharides, water and either or both of DHA (dihydroxyacetone) and MGO (methylglyoxal) and having at least one further characteristic being: a pH of 3.0 to 5.0; hydrogen peroxide; phenolics characteristic of active honeys; bee defensins, catalase enzyme; a water activity less than 0.7; or
   (iv) combinations of the honey, the honey fraction and the honey analogue,
wherein said active honey is a honey with an antimicrobial activity in the absence of hydrogen peroxide, and wherein the honey or honeys used or from which the honey fraction is derived include those derived from plants of the *Leptospermum* species.

Described herein is a method of inhibiting yeast growth by administrating a composition substantially as described above to a subject in need thereof.

Also described herein is a yeast inhibitive wound dressing including a composition substantially as described above.

Also described herein is a yeast inhibitive aqueous based medicament including a composition substantially as described above.

The inventor's have unexpectedly found that the combination of honey, a honey fraction or a honey analogue along with lactoferrin has a synergistic effect on inhibiting yeast growth. This inhibition effect is far greater than the individual components themselves. This synergism is not predictable from the art and appears to relate to an interaction between compounds or characteristics of honey and lactoferrin. The inhibition effect appears to be highly synergistic. For example, only very dilute quantities of honey and lactoferrin are required to achieve inhibition and the inhibition effect goes beyond what either of the components achieve alone.

Advantages of the described combination will become apparent. However, the effects show considerable promise in the treatment of yeast infection (external and internal) and may provide an alternative to treatment with drugs.

### BRIEF DESCRIPTION OF THE DRAWINGS

Further aspects of the application will become apparent from the following description that is given by way of example only and with reference to the accompanying drawings in which:
- Figure 1: shows a graph illustrating *Candida albicans* growth when subjected to varying concentrations of honey in solution;
- Figure 2: shows a graph illustrating *Candida albicans* growth when subjected to varying concentrations of lactoferrin in solution;
- Figure 3: shows a graph illustrating *Candida albicans* growth when subjected to varying concentrations of honey in combination with lactoferrin (5mg/ml) in solution;
- Figure 4: shows a graph illustrating *Candida albicans* growth when subjected to two different lactoferrin (0.2%, 0.6%) concentrations [Lac=Lactoferrin; Cat=Catalase. n=3, error bars represent SD];
- Figure 5: shows the above results of Figure 4 expressed in terms of percentage inhibition as a proportion of media growth alone [Lac=lactoferrin; Cat=catalase. n=3 per control and treatment];
- Figure 6: shows a graph illustrating *Candida albicans* growth when subjected to varying concentrations of lactoferrin in solution [(cat=catalase; lac=lactoferrin]; and
- Figure 7: shows a graph illustrating *Candida albicans* growth when subjected to varying concentrations of honey along with lactoferrin in solution also testing a honey analogue composition [AH=artificial honey; MGO=methylglyoxal, cat=catalase; lac=lactoferrin].

### DETAILED DESCRIPTION

As noted above, the application broadly relates to the combination of honey, an active fraction of honey or a honey analogue with lactoferrin as a means to inhibit the growth of yeasts.

For the purposes of this specification, the term 'honey' refers to naturally produced honey (i.e. produced by bees) containing at least a mix of glucose, fructose, water and glucose oxidase enzyme.

The term 'honey analogue' refers to a mixture of 30-50% glucose, 30-50% fructose, 1-18% water and either or both of glucose oxidase enzyme and/or hydrogen peroxide. Where the analogue is used shortly after production, hydrogen peroxide itself may be used. Where the analogue may be stored for a period of time, the analogue by preference contains glucose oxidase enzyme.

As may be appreciated, glucose oxidase enzyme converts sugars into hydrogen peroxide that also results in a lower pH. If hydrogen peroxide alone is used and then the analogue stored, it is possible that the peroxide level will decrease by a normal reduction equilibrium and the pH level then increase. Using glucose oxidase enzyme ensures a steady level of hydrogen peroxide and hence steady pH. The quantities used are intended to approximate the composition of naturally produced honey.

The term 'honey fraction' refers to a naturally produced honey where substantially all of the monosaccharide portion of the honey has been removed to produce a honey fraction. The monosaccharide portion may include fructose and glucose. Honey fractions referred to in this specification include a UMF or non-peroxide activity containing portion of the honey as well as other non-saccharide components including compounds selected from: phenolics, bee defensins, and catalase enzyme.

The term 'chelator' and grammatical variations thereof refer to compounds or substances containing compounds that act to scavenge and bind metal ions in a solution.

The term 'active honey' refers to a honey with an antimicrobial activity in the absence of hydrogen peroxide referred to interchangeably as a honey containing UMF activity, MGO activity or containing DHA compound.

The term 'Unique Manuka Factor', 'UMF' and non-peroxide activity' refer to the activity of honey not directly attributable to the antimicrobial effects of honey conferred from the normal honey pH and osmolarity, for example that observed and measured in a naturally derived clover honey. The term 'UMF' may be used interchangeably with methylglyoxal (MGO) concentration or dihydroxyacetone (DHA) concentration since MGO is known to be the compound attributable to UMF activity and DHA is a precursor compound of MGO also present in UMF honey and attributable to the UMF activity.

The term 'gelling agent' or grammatical variations thereof refers to an agent that, in the absence of liquid is not a gel, but the agent is able to form a gel in the presence of liquid.

The term 'dressing' refers to any covering that may be applied to a lesion where lesions encompass infected and non-infected abrasions, cuts, bits, burns, wounds, ulcers, abscesses, surgical wounds, fungating tumours and pressure sores.

The term 'bioactive' refers to the composition containing biologically active compounds that inhibit yeast growth.

The term 'therapeutically effective' with reference to an amount or dosage of a composition or medicament noted refers to an amount of a composition that is sufficient to effectively inhibit yeast growth.

The term 'about' refers to a quantity, level, value, number, frequency, percentage, dimension, size, weight, or length that varies by as much as 30, 25, 20,15, 10,9, 8, 7, 6, 5, 4, 3, 2 or 1% to a reference quantity, level, value, number, frequency, percentage, dimension, size, weight, or length.

The term 'natural based' refers to compounds obtained from nature or one or more synthesised versions of compounds found in nature. Ideally, the compound or compounds used meet the Natural Products Association (NPA) guidelines i.e. they are derived from renewable sources in nature' they do not use petroleum compounds, they meet generally recognised as safe or GRAS standard as set by the USA FDA and they are manufactured based on NPA approved processes.

Described herein is a yeast inhibitive composition including:
a. a therapeutically effective amount of at least one active honey; and
b. a therapeutically effective amount of lactoferrin.

Also described herein is a yeast inhibitive composition including:
a. a therapeutically effective amount of at least one bioactive fraction of active honey wherein the monosaccharides of the honey have been removed; and
b. a therapeutically effective amount of lactoferrin.

Also described herein is a yeast inhibitive composition including:
a. a therapeutically effective amount of at least one honey analogue containing monosaccharides, water and either or both of DHA and MGO and having at least one further characteristic being: a pH of 3.0 to 5.0; phenolics characteristic of active honeys; bee defensins, a water activity less than 0.7; and
b. a therapeutically effective amount of lactoferrin.

The inventors have unexpectedly found that the combination of honey, a honey fraction or a honey analogue along with lactoferrin has a synergistic effect on inhibiting yeast growth. This inhibition effect is far greater than the individual components themselves. This synergism is not predictable from the art and appears to relate to an interaction between compounds or characteristics of the honey and lactoferrin. The inhibition effect appears to be highly synergistic. For example, only very dilute quantities of honey and lactoferrin are required to achieve inhibition and the inhibition effect goes beyond what either of the components achieve alone.

Microbial growth may be inhibited by at least about 20% over a 24-hour time period as measured via optical density. Microbial growth may be inhibited by at least about 40% over a 24-hour time period as measured via optical density. Microbial growth may be inhibited by at least about 60% over a 24-hour time period as measured via optical density. Microbial growth may be inhibited by at least about 80% over a 24-hour time period as measured via optical density. Microbial growth may be inhibited by 100% over a 24-hour time period as measured via optical density.

The yeast may be from the genus *Candida.* The yeast may be a strain of *Candida albicans.* While many *Candida* species are comparatively benign, some *Candida* may be harmful and some that are normally benign may become problematic if the host immune system is comprised or if the *Candida* infects the wrong location. *Candida albicans* in particular may be problematic and is associated with candidiasis or thrush in humans and other animals especially in immune-compromised patients. Literature suggests that systemic infections of the bloodstream and major organs with *Candida* yeasts particularly in immune-comprised patients affect over 90,000 people a year in the USA with a 40-50% mortality. *Candida* infection is therefore a key issue. Overgrowth of several species of *Candida* including *albicans* can cause superficial infections such as oropharyngeal candidiasis (thrush) and vulvovaginal candidiasis (vaginal Candidiasis). Oral candidiasis is common in elderly denture wearers.

In otherwise healthy individuals, *Candida* infections can be cured with topical or systemic antifungal medications (commonly over-the-counter treatments like miconazole or clotrimazole). Miconazole may have unwanted side effects from drug interactions as it may be absorbed into the intestinal tract when used orally. Clotrimazole may also have drug interactions and a range of other side effects including skin rash, hives, blistering, burning, itching, peeling, redness, stinging, swelling, or other sign of skin irritation. Use of these over the counter treatments can therefore be problematic. In debilitated or immune-compromised patients, or if introduced intravenously, candidiasis may become a systemic disease producing abscess, thrombophlebitis, endocarditis, or infections of the eyes or other organs.

As should be appreciated, the composition described using honey and lactoferrin uses two natural based products that are relatively benign and have minimal if any side effects offering a significant advantage over art treatments.

Use of honey to treat *Candida* is somewhat counter intuitive as *Candida* ferment glucose and other sugars to acid and gas, sucrose to acid. Since sugars are key components in honey, use of honey would be expected to encourage *Candida* growth as opposed to inhibit growth.

The honey used or honey from which the honey fraction is derived from may have both peroxide and non-peroxide activity i.e. it is an 'active' honey.

Peroxide activity stems from the activity of the enzyme glucose peroxidase present in all honeys. The enzyme originates from bees and catalyses the production of hydrogen peroxide upon dilution of the honey, for example when placed on a wound.

Non-peroxide activity is often used interchangeably with the measurement of 'unique manuka factor' or 'UMF' activity and more recently MGO concentration and refers to the honey retaining its antimicrobial activity in the absence of the production of hydrogen peroxide.

The honey used may have the compounds dihydroxyacetone (DHA), methylglyoxal (MGO) or both present. Recent research has found the presence of DHA and MGO in 'active' honeys such as manuka honey. DHA appears to be a precursor for MGO and MGO appears to contribute to the anti-microbial effects of active honeys.

Honey has inherent physical characteristics such as low pH and osmolarity that deter microbial growth. However, honeys with non-peroxide activity are often desired or preferred in medical applications. This is because of their enhanced anti-microbial effects and as a result may be termed 'active' honeys.

Suitable honeys include those derived from plants of the *Leptospermum* species. The term *'Leptospermum* species' refers to a genus of plants in the myrtle family Myrtaceae and includes: *Leptospermum scoparium, Leptospermum polygalifolium, Leptospermum semibaccatum, Leptospermum semibaccatum, Leptospermum trinervium, Leptospermum whitei, Leptospermum speciosum* and *Leptospermum liversidgei.* The phrase 'derived from a *Leptospermum* sp.' refers to a honey that is produced from nectar, at least about 50%, for example 75%, 85%, 95% or 98%, of which is derived from one or more *Leptospermum* species.

Also as noted in the definition above, although the term 'honey' has been used, a honey analogue containing MGO and other compounds or properties characteristic of honey may be used. Alternatively, a honey fraction with the monosaccharide portion removed may equally be used. The phrase 'removed' used above refers to a honey fraction containing less than at least about 50%, for example 75%, 85%, 95% or 98% of the saccharide concentration that would be found in the unfractionated honey.

The inventors have unexpectedly found that comparatively small concentrations of honey or lactoferrin are required to achieve a yeast inhibitory effect when used in combination. The concentrations required are well below what might be expected from the individual components.

The composition may contain honey, honey analogue or honey fraction at a concentration of at least 1% w/v in the composition. The concentration may be at least 2%, for example, at least 3%, 4%, 5%, 6%, 7%, 8%, 9%, 10%, 12%, 15%, 18%, 20% w/v in the composition.

Those skilled in the art should appreciate that the above concentrations are very low compared to what might be expected to inhibit yeast growth. Without the lactoferrin present, honey concentrations well above 20% w/v would likely be necessary to even begin to inhibit yeast growth. The order of magnitude difference conferred by the use of the combination of honey and lactoferrin is therefore highly unexpected.

The composition includes lactoferrin, being a compound or compounds that scavenge free transition metal ions, specifically iron in the case of lactoferrin. Lactoferrin is known to inhibit yeast growth but in the inventor's experience, not to the extent demonstrated in from the combination with honey as described herein.

As noted above, only small concentrations of lactoferrin are required in the combination in order to confer a substantial inhibitory effect. By contrast, lactoferrin alone does not completely inhibit yeast growth.

The composition may include at least 0.1 mg/ml (0.1% w/v) lactoferrin. The lactoferrin concentration may be at least 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1 mg/ml, 2 mg/ml, 3 mg/ml, 4 mg/ml, 5 mg/ml, 6 mg/ml, 7 mg/ml, 8 mg/ml, 9 mg/ml or 10 mg/ml (or % w/v).

It should be noted that lactoferrin may be used in a concentrated isolate form. Lactoferrin may also be used in an un-concentrated or dilute form such as from milk, colostrum, a milk concentrate or partial lactoferrin concentrate from a raw material source, or from a synthetic source (purified or non-purified form).

The honey analogue if used, may contain further characteristics of active honey. The inventors have found that an analogue with MGO and lactoferrin does not inhibit yeast growth to the same extent as a combination of active honey and lactoferrin. Modification of the honey analogue to include other characteristics of active honey may be completed to enhance the inhibitive effect. Such characteristics to include may be selected from: use of a reduced pH, fortification with one or more phenolic compounds, fortification with bee defensins, use of a water activity of less than 0.7. It is the inventor's understanding however that hydrogen peroxide is not a contributing factor to the measured inhibitive effects. This has been confirmed in trials where the hydrogen peroxide content was knocked out via the use of catalase enzyme. The resulting inhibition was unchanged whether hydrogen peroxide was present or not.

The composition may contain one or more additional phenolic compounds. The honey analogue if used may be fortified with one or more phenolic compounds.

The phenolic compounds may be selected from: phenolic acids, phenolic salts, phenolic esters, related polyphenolic compounds, and combinations thereof.

The phenolic compounds may be methoxylated. By way of example, the applicant has analysed the phenolics prominent in manuka *(Leptospermum* spp.) and a large number of these phenolics are methoxylated at one or more points of their phenol or acid group. Compounds such as gallic or benzoic acid may be present mainly in their methoxylated form such as methoxybenzoic acid, methoxygallic acid, methyl syringate, methoxyphenylactic acid or syringic acid. Methoxylated phenolics may represent greater than for example 10%, 20% or 30% wt of the total phenolic compound content in the composition or contain at least 150 mg/kg of methoxylated phenolic compounds.

The phenolic compounds may be selected from: phenyllactic acid, methoxylated phenyllactic acid, methoxylated benzoic acids, syringic acid, methyl syringate, isomeric forms of methyl syringate, and combinations thereof.

Phenolic compounds may be present at a concentration in the composition of 5mg/kg to 10,000mg/kg.

The phenolic compounds used may be selected from: 2-methoxybenzoic acid and 4-methoxybenzoic acid.

Fortification of the honey, honey fraction or honey analogue may be completed to achieve the above concentrations and/or to introduce one or more of the above types of phenolic compound.

The composition may include a carrier. The carrier may be selected to suit the intended formulation or method of administration.

A wide variety of carriers or vehicles may be used. In one embodiment the at least one carrier or vehicle has a melting point of about 37°C or greater.

In one embodiment, the carrier may comprises from about 1% to about 99.9% wt of the composition.

The composition may be in a form selected from: a liquid, a gel, a cream, an ointment, a wound dressing, a solid form tablet or capsule, and combinations thereof.

The carrier may be water. Water may be a useful carrier for liquid formulations or for encapsulated formulations.

The carrier may be a powder or granular substance into which the composition is incorporated, for example for administration via a tablet or capsule.

The above carriers may be selected based on whether the composition is to be formulated for internal use or external use.

Described herein is a method of inhibiting yeast growth by administrating a composition substantially as described above to a subject in need thereof.

As should be appreciated, the composition may have a variety of treatment applications to inhibit yeast growth and thereby allow other treatment measures to take effect. For example, the composition may be applied to a wound, skin or mucus lining to inhibit yeast growth, while the patient is co-administered a traditional anti-fungal remedy. This dual approach avoids the region becoming further infected, slows spread of the infection and allows time for the traditional anti-fungal remedy to take effect.

The composition may be administered topically to a subject. Topical treatments using honey based dressings and formulations are well known in the art and the combination described herein provides an added and potentially more potent method of treatment. In one envisaged application, the composition may be an aqueous based medicament such as a cream or gel that is rubbed onto the skin or mucus lining(s) of the subject to directly treat an infection or instead, to prevent secondary infections related to fungi or yeast.

The subject may be a human. Alternatively, the subject may be a non-human animal. As should be appreciated, humans and animals can equally be treated using the yeast inhibitory composition as the physiology of a yeast based infection may be similar between humans and at least other mammals. Non-limiting examples of animals to which the composition may be administered include horses, livestock including cattle, sheep and deer and companion animals such as cats and dogs.

Described herein is a yeast inhibitive wound dressing including a composition substantially as described above.

Also described herein is a yeast inhibitive aqueous based medicament including a composition substantially as described above.

As should be appreciated, wound dressings and aqueous based medicaments incorporating honey are well known and researched. Examples include those described in at least US7,714,183, US6,956,144, US11/106,473, US12/091,897 and US12/301,931. The yeast inhibitive combination described herein and the synergies that the combination provides in inhibiting yeast growth have considerable power to improve current wound dressings and medicaments.

The dressing or aqueous based medicament may include at least one gelling agent. As noted above and in the art, gelling agents are advantageous for use with honey for wound applications. In particular, the gelling agents reduce the tackiness of the honey, yet provide a more cohesive structure such as a sheet structure or viscous gel that is easier to apply to a wound, skin region or mucosal lining. Gelling agents also have the advantage that they may be absorbent and work to move exudate away from a wound environment. This consequently avoids dilution of the honey and lactoferrin at the site.

The gelling agent may be selected from: an absorbent synthetic polymer, an absorbent natural based polymer, and combinations thereof.

The absorbent synthetic polymer may be selected from: any cross-linked sodium polyacrylate, polyacrylamide copolymer, ethylene maleic anhydride copolymer, carboxymethyl cellulose, polyvinyl alcohol copolymer, isobutylene-maleic anhydride copolymer, cross-linked polyethylene oxide, starch grafted copolymer or polyacrylonitrile, gauze, and combinations thereof.

The absorbent natural based polymer may be selected from: alginate, agar, natural based gums, and combinations thereof.

In the above embodiment where alginate is used, the alginate may be selected from: calcium alginate, sodium alginate, and combinations thereof.

The gelling agents may be present as a powder, granule or fibre.

As noted above, other pharmaceutical antimicrobial agents may be added to the described composition and/or co-administered. One example envisaged is the co-administration of clotrimazole anti-fungal agent with the composition of the above embodiments. In this way, irrespective of any potential synergies, the user need only administer a single composition rather than multiple compositions.

In the above embodiments, the composition may be irradiated with a sterilisation effective dose of radiation. Typically, the dose may be for example, 5kGy, 10kGy, 15kGy, 20kGy, 25kGy or 30kGy, the amount dependent on the end application and what is required in order to sufficiently sterilise the composition and meet market regulatory approvals.

The aspects described above may also be said broadly to consist in the parts, elements and features referred to or indicated in the specification of the application, individually or collectively, and any or all combinations of any two or more said parts, elements or features, and where specific integers are mentioned herein which have known equivalents in the art to which the embodiments relate, such known equivalents are deemed to be incorporated herein as if individually set forth,

Where specific integers are mentioned herein which have known equivalents in the art to which this invention relates, such known equivalents are deemed to be incorporated herein as if individually set forth.

Advantages of this combination will become apparent however, the effects show considerable promise in the treatment of yeast infection (external and internal) and may provide an alternative to treatment with antibiotics or other drugs.

### WORKING EXAMPLES

The composition, methods and uses are now described with reference to examples illustrating embodiments of the composition.

### EXAMPLE 1

In this example, a manuka honey was mixed with lactoferrin at varying concentrations and the degree of *Candida albicans* growth was measured and compared to each of the individual components alone.

Samples of honey alone being a manuka honey with a UMF rating of ∼15 were collected. The honey was diluted with distilled water to honey concentrations of 12.5, 11.25, 10, 8.75, 7.5 and 0 %(w/v).

Samples of lactoferrin alone were also collected and similarly diluted with distilled water to concentrations of 5mg/mL (5%), 4.5mg/mL(4.5%), 4mg/mL (4%), 3.5mg/mL (3.5%), 3mg/mL (3%) and 0% (w/v).

Mixed samples were then produced using honey concentrations of 12.5, 11.25, 10, 8.75, 7.5 and 0 %(w/v) along with lactoferrin at a concentration of 5mg/mL of lactoferrin.

Four replications were completed for each of the samples.

Microbial growth for each of the samples was then measured every 2hrs continuously for up to 24hrs, determined by measuring the optical density of the mixture.

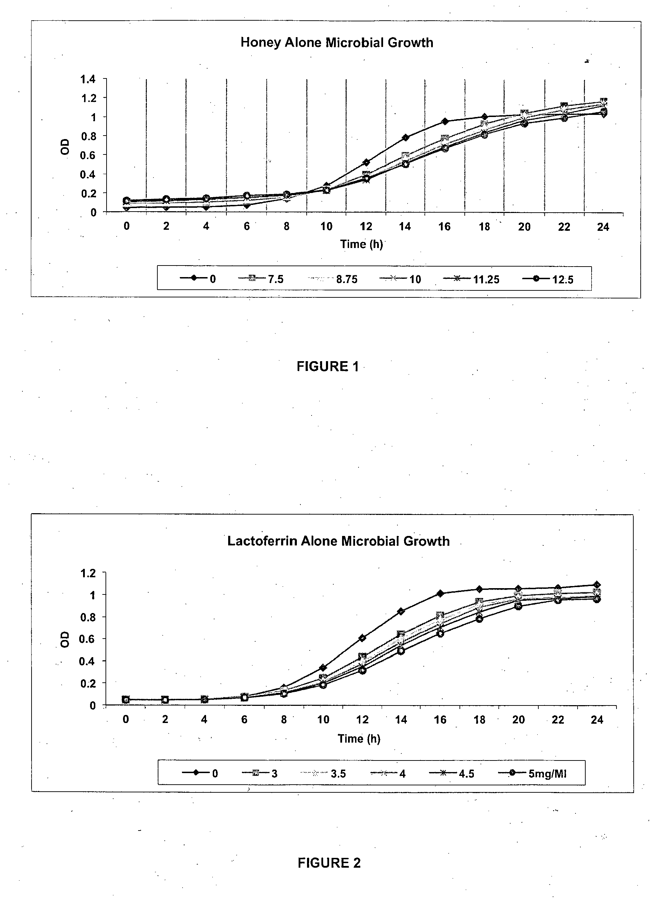

As shown in Figures 1 and 2, honey and lactoferrin both inhibit microbial growth compared to not having honey or lactoferrin present at all. However, at the high dilutions / low concentrations tested, the degree of inhibition was relatively low. Further, after 24 hours, there was little difference observed between samples with honey or lactoferrin compared to the controls without honey or lactoferrin.

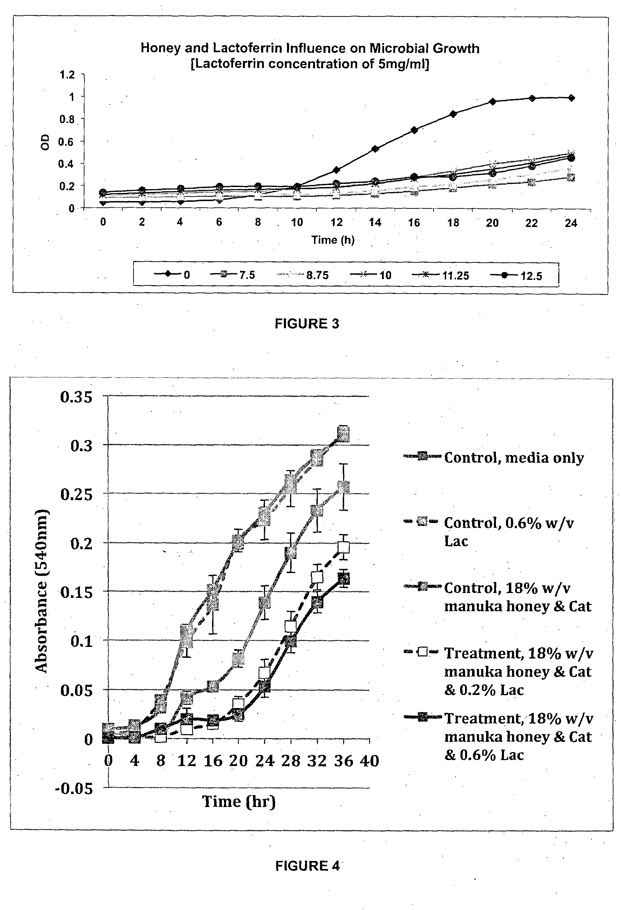

When the honey and lactoferrin were combined (Figure 3), the results were dramatically different with a greatly decreased degree of inhibition. After 24 hours, the degree of inhibition was only 20-40% of that observed in the control with no honey or lactoferrin present. The combination of honey and lactoferrin consequently inhibited microbial growth by 60-80% over a 24 hour time period.

These results were furthermore surprising, as even with the lowest concentration of honey 7.5% w/v, the inhibitory effect was observed. This suggests that the combination is very potent at inhibiting microbial growth even at very low concentrations

### EXAMPLE 2

A further experiment was completed measuring the growth curves of *Candida albicans* grown at 28°C measured by absorbance (540nm). As shown in Figure 4, the synergistic effect of lactoferrin at 0.2% and 0.6% w/v is illustrated when used in conjunction with 18% w/v manuka honey and catalase. Catalase was added to remove any confounding effects from hydrogen peroxide. As may be appreciated, the peroixde effect can vary between and within honeys giving rise to poor results that are difficult to interpret. Removal of the peroxide effect gives clearer result.

It should be noted that a lower growth temperature than usual was used to prevent filamentous growth, which is an artifact of these *Candida* assays. The results found were clean easily measurable results and were verified under a microscope to be accurate.

### EXAMPLE 3

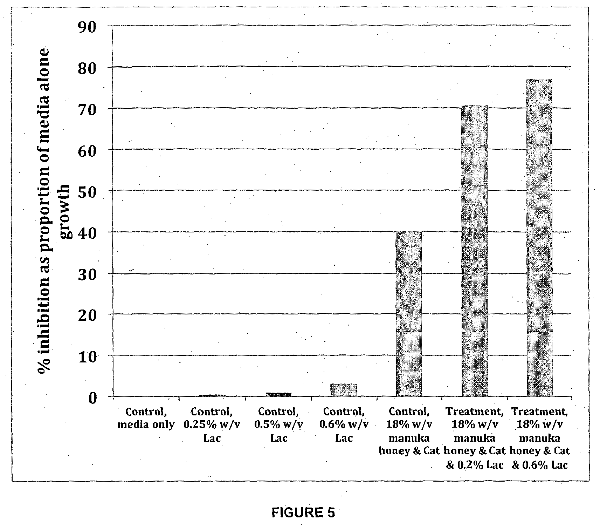

A further experiment was completed measuring the percentage inhibition expressed as a proportion of *Candida albicans* growth in media alone. As shown in Figure 5, the results further demonstrated the negligible effects of lactoferrin used in isolation against the synergistic effect (double the inhibition) of lactoferrin at 0.2% and 0.6% w/v when used in conjunction with 18% w/v manuka honey and catalase. Catalase was added to remove the peroxide effect noted above.

### EXAMPLE 4

A further set of *Candida albicans* repeat trials were completed where the growth of *Candida albicans* cultures in broth was measured when subjected to a 10% w/v manuka honey with 1 mg/ml catalase and lactoferrin treatments ranging from 2.5-10 mg/ml. To prevent filamentous growth, the assay temperature was 28°C as used previously. The assay was a broth microtitre plate set-up. Growth was measured by absorbance at 650nm. Catalase was added to remove the peroxide effect noted above.

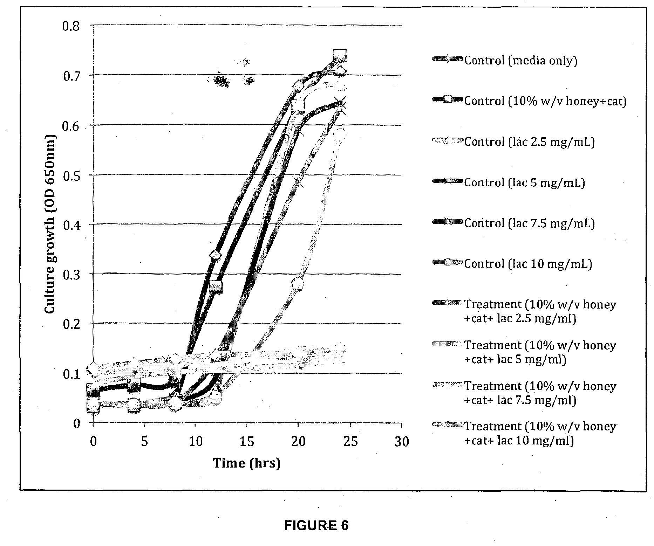

Figure 6 confirms the combination inhibition (almost 100% inhibition) and minimal inhibition with honey alone or lactoferrin alone. Figure 6 illustrates the results using a 10% w/v honey dilution. Similar repeats were also completed for 5% w/v and 15% w/v honey dilutions and these gave the same resutls as that shown in Figure 6 for a 10% w/v dilution.

### EXAMPLE 5

In this example, the use of a honey analogue was compared to various controls and honey and lactoferrin combinations. The growth of *Candida albicans* cultures in broth were subject to 10% w/v manuka honey with 1 mg/ml catalase treatment, artificial honey (honey analogue) and methylglyoxal (MGO) treatments relative to the honey treatment, and lactoferrin treatments ranging from 7.5-10 mg/ml. Catalase was added to remove the peroxide effect noted above.

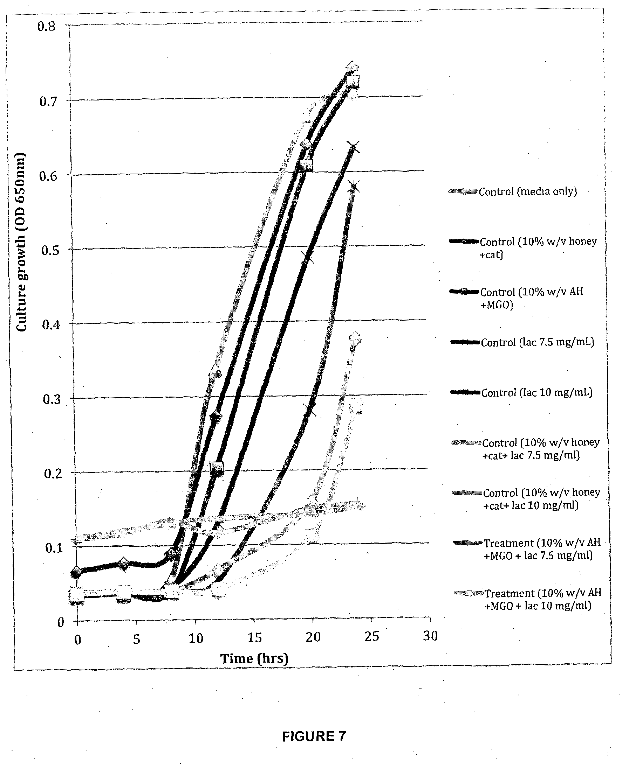

The results as shown in Figure 7 confirm the synergy of the combination i.e. virtually 100% inhibition.

The honey analogue used was a sugar syrup containing MGO equivalent to that found in the manuka honey used in the trial. The honey analogue did not achieve the same degree of inhibition as the honey and lactoferrin combination tested even when used with comparative concentrations of lactoferrin. This illustrates that the synergism is in part due to aspects such as peroxide activity, osmolarity and MGO content but also requires another characteristic seen in the combination of manuka honey and lactoferrin. Characteristics that may be attributable to the full inhibition effects observed for active honey beyond just MGO and lactoferrin alone include: the pH of honey, the presence of hydrogen peroxide, the presence of one or more phenolic compounds, the presence of bee defensins or a water activity of 0.7.

### EXAMPLE 6

In this example, the compositions described in earlier examples are administered to treat a topical wound or yeast infection of a mucus lining.

A composition may be manufactured by preparing any one of the mixtures as follows:
Mixture 1 : manuka honey and lactoferrin
Mixture 2:jellybush honey and lactoferrin
Mixture 3: a honey analogue including glucose, fructose, peroxidase enzyme, DHA and MGO, tannins extracted from manuka honey along with lactoferrin
Mixture 4: a non-peroxide activity containing fraction of honey with 95% of all monosaccharides removed with lactoferrin

The mixtures are applied to a wound on a human or an animal e.g. a horse. Optionally, the mixtures may be formulated as per existing wound dressings e.g. those described in US 7,714,183, US 6,956,144, US 11/106,473, US 12/091,897, US 12/301,931.

## Claims

1. A composition for use in the therapeutic inhibition of yeast growth, the composition including:
a. a therapeutically effective amount of lactoferrin; and
b. a therapeutically effective amount of either:
i. at least one active honey; or
ii. at least one bioactive fraction of active honey wherein the monosaccharides of the honey have been removed; or
iii. at least one honey analogue containing monosaccharides, water and either or both of dihydroxyacetone and MGO methylglyoxal and having at least one further characteristic being: a pH of 3.0 to 5.0; hydrogen peroxide; phenolics characteristic of active honeys; bee defensins, catalase enzyme; a water activity less than 0.7; or
iv. combinations of the honey, the honey fraction and the honey analogue,
wherein said active honey is a honey with an antimicrobial activity in the absence of hydrogen peroxide, and wherein the honey or honeys used or from which the honey fraction is derived include those derived from plants of the *Leptospermum* species.

2. The composition for use as claimed in claim 1 wherein the composition includes at least 1% w/v honey, honey fraction or honey analogue.

3. The composition for use as claimed in any one of the above claims wherein the honey analogue is fortified with at least one phenolic compound.

4. The composition for use as claimed in claim 3 wherein the phenolic compound or compounds are selected from: phenyllactic acid, methoxylated phenyllactic acid, methoxylated benzoic acids, syringic acid, methyl syringate, isomeric forms of methyl syringate, and combinations thereof.

5. The composition for use as claimed in any one of the above claims wherein the yeast is from the genus *Candida.*

6. The composition for use as claimed in any one of the above claims wherein the yeast is a strain of *Candida albicans.*

7. The composition for use as claimed in any one of the above claims wherein the composition includes at least 0.1mg/ml (0.1% w/v) lactoferrin.

8. The composition for use as claimed in any one of the above claims wherein the composition includes a carrier.

9. The composition for use as claimed in any one of the above claims wherein the composition is formulated for topical application to a wound, skin or mucus lining.

10. The composition for use as claimed in any one of the above claims wherein the composition is for use by co-administration of the composition and a separate anti-fungal agent or agents.

## Patentansprüche

1. Zusammensetzung zur Verwendung bei der therapeutischen Hemmung von Hefewachstum, wobei die Zusammensetzung Folgendes umfasst:
a. eine therapeutisch wirksame Menge Lactoferrin; und
b. eine therapeutisch wirksame Menge von einem der Folgenden:
i. zumindest einem aktiven Honig; oder
ii. zumindest einer bioaktiven Fraktion von aktivem Honig, wobei die Monosaccharide des Honigs entfernt wurden; oder
iii. zumindest einem Honiganalogon, das Monosaccharide, Wasser und eines oder beide aus Dihydroxyaceton und MGO Methylglyoxal enthält und zumindest ein weiteres der folgenden Merkmale aufweist: einen pH von 3,0 bis 5,0; Wasserstoffperoxid; Phenoleigenschaften von aktivem Honig; Bienen-Defensine, Katalaseenzym; eine Wasseraktivität unter 0,7; oder
iv. Kombinationen aus dem Honig, der Honigfraktion und dem Honiganalogon,
wobei der aktive Honig ein Honig mit antimikrobieller Aktivität in Abwesenheit von Wasserstoffperoxid ist und wobei der Honig oder die Honige, der/die verwendet wird/werden oder von dem/denen die Honigfraktion abgeleitet ist, jene umfasst/umfassen, die von Pflanzen der Spezies Leptospermum stammen.

2. Zusammensetzung zur Verwendung nach Anspruch 1, wobei die Zusammensetzung zumindest 1 % (Gew./Vol.) Honig, Honigfraktion oder Honiganalogon umfasst.

3. Zusammensetzung zur Verwendung nach einem der vorangegangenen Ansprüche, wobei das Honiganalogon mit zumindest einer Phenolverbindung verstärkt ist.

4. Zusammensetzung zur Verwendung nach Anspruch 3, wobei die Phenolverbindung oder -verbindungen aus Folgenden ausgewählt ist/sind: Phenylmilchsäure, methoxylierter Phenylmilchsäure, methoxylierten Benzoesäuren, Syringasäure, Methylsyringat, isomeren Formen von Methylsyringat und Kombinationen davon.

5. Zusammensetzung zur Verwendung nach einem der vorangegangenen Ansprüche, wobei die Hefe von der Gattung Candida ist.

6. Zusammensetzung zur Verwendung nach einem der vorangegangenen Ansprüche, wobei die Hefe ein Stamm von Candida albicans ist.

7. Zusammensetzung zur Verwendung nach einem der vorangegangenen Ansprüche, wobei die Zusammensetzung zumindest 0,1 mg/ml (0,1 % (Gew./Vol.)) Lactoferrin enthält.

8. Zusammensetzung zur Verwendung nach einem der vorangegangenen Ansprüche, wobei die Zusammensetzung einen Träger umfasst.

9. Zusammensetzung zur Verwendung nach einem der vorangegangenen Ansprüche, wobei die Zusammensetzung zur topischen Aufbringung auf eine Wunde, auf Haut oder auf Schleimhautauskleidung formuliert ist.

10. Zusammensetzung zur Verwendung nach einem der vorangegangenen Ansprüche, wobei die Zusammensetzung zur Verwendung durch gemeinsame Verabreichung der Zusammensetzung und eines oder mehrerer separater Antimykotika dient.

## Revendications

1. Composition à utiliser dans l'inhibition thérapeutique de la croissance de levure, la composition comprenant :
a. une quantité thérapeutiquement efficace de lactoferrine ; et
b. une quantité thérapeutiquement efficace de :
i. au moins un miel actif ; ou
ii. au moins une fraction bioactive de miel actif, dans laquelle les monosaccharides du miel ont été éliminés ; ou
iii. au moins un analogue de miel contenant des monosaccharides, de l'eau et/ou de la dihydroxyacétone et du méthylglyoxal, MGO, et ayant au moins une autre caractéristique étant : un pH de 3,0 à 5,0 ; du peroxyde d'hydrogène ; des phénoliques caractéristiques de miels actifs ; des défensines d'abeilles, une enzyme catalase ; une activité hydrique inférieure à 0,7 ; ou
iv. des combinaisons du miel, de la fraction de miel et de l'analogue de miel,
dans laquelle ledit miel actif est un miel ayant une activité antimicrobienne en l'absence de peroxyde d'hydrogène, et dans laquelle le miel ou les miels utilisés ou à partir desquels la fraction de miel est dérivée comprennent ceux dérivés de plantes de l'espèce *Leptospermum.*

2. Composition à utiliser selon la revendication 1, dans laquelle la composition comprend au moins 1 % p/v de miel, de fraction de miel ou d'analogue de miel.

3. Composition à utiliser selon l'une quelconque des revendications précédentes, dans laquelle l'analogue de miel est enrichi avec au moins un composé phénolique.

4. Composition à utiliser selon la revendication 3, dans laquelle le ou les composés phénoliques sont choisis parmi : acide phényllactique, acide phényllactique méthoxylé, acides benzoïques méthoxylés, acide syringique, syringate de méthyle, formes isomères de syringate de méthyle, et des combinaisons de ceux-ci.

5. Composition à utiliser selon l'une quelconque des revendications précédentes, dans laquelle la levure provient du genre *Candida.*

6. Composition à utiliser selon l'une quelconque des revendications précédentes, dans laquelle la levure est une souche de *Candida albicans.*

7. Composition à utiliser selon l'une quelconque des revendications précédentes, dans laquelle la composition comprend au moins 0,1 mg/ml (0,1 % p/v) de lactoferrine.

8. Composition à utiliser selon l'une quelconque des revendications précédentes, dans laquelle la composition comprend un support.

9. Composition à utiliser selon l'une quelconque des revendications précédentes, dans laquelle la composition est formulée pour une application topique sur une plaie, une peau ou une muqueuse.

10. Composition à utiliser selon l'une quelconque des revendications précédentes, dans laquelle la composition est à utiliser par co-administration de la composition et d'un ou plusieurs agents antifongiques séparés.
